# NEW EUROPEAN PATENT SPECIFICATION

(11) **EP 2 859 891 B2**
(45) Date of publication and mention of the opposition decision: **06.10.2021**
(45) Mention of the grant of the patent: 21.02.2018
(21) Application number: 14187915.5
(22) Date of filing: 07.10.2014
(51) Int. Cl.: A61K 31/198, A61K 31/401, A61K 47/32, A61K 31/717, A61K 31/728, A61K 47/36, A61K 47/38, A61K 9/00, A61P 1/04

(54) **Use of a combination for the prevention of radiation- or chemotherapy-induced mucositis**
Verwendung einer Kombination zur Vorbeugung von Strahlungs- oder Chemotherapie-induzierter Mukositis
Utilisation d'une combinaison pour la prévention de la mucite induite par la radio ou la chimiothérapie

(30) Priority: 08.10.2013 IT MI20131660
(43) Date of publication of application: 15.04.2015
(73) Proprietor: Professional Dietetics S.p.A. in forma abbreviata P.D. S.p.A., 20129 Milan (IT)
(72) Inventor: Giorgetti, Paolo, 20129 MILANO (IT)
(74) Representative: Minoja, Fabrizio

(56) References cited:
- WO-A1-2004/103383
- WO-A1-2011/064297
- WO-A1-2011/064297
- US-A1- 2005 256 042
- Cirillo N: "Prevention of fibroblast senescence by hyaluronic acid and clinical application to chemoradiationinducedoral mucositis", INTERNET , 24 September 2013 (2013-09-24), XP002724225, Retrieved from the Internet: URL:http://www.omicsgroup.com/conferences/ cancer-science-therapy-2013/ [retrieved on 2014-05-12]
- Silvano G: "Prevenzione e gestione di mucosite, dolore ed emesi", INTERNET , 15 December 2012 (2012-12-15), XP002724227, Retrieved from the Internet: URL:http://www.congressiairo.it/2012/taran to10anni/SOLOPERTO.pdf [retrieved on 2014-05-12]
- Menotti C: "Mucosamin spray", INTERNET , 4 November 2010 (2010-11-04), XP002724226, Retrieved from the Internet: URL:http://www.solartium-dietetics.com/sol artium/sites/default/files/u9/mucosamin.pd f [retrieved on 2014-05-12]
- LIONEL D'HONDT ET AL: "Oral mucositis induced by anticancer treatments: physiopathology and treatments.", THERAPEUTICS AND CLINICAL RISK MANAGEMENT JUN 2006, vol. 2, no. 2, June 2006 (2006-06), pages 159-168, ISSN: 1176-6336
- Colella G; Cannavale R; Vicidomini A; Rinaldi G; Compilato D; Campisi G: "Efficacy of a spray compound containing a pool of collagen precursor synthetic aminoacids (l-proline, l-leucine, l-lysine and glycine) combined with sodium hyaluronate to manage chemo/radiotherapy-induced oral mucositis: preliminary data of an open trial", International Journal of Immunopathology and Pharmacology,, vol. 23, no. 1, 2010, pages 143-151,
- CIRILLO N: "Prevention of fibroblast senescence by hyaluronic acid and clinical application to chemoradiationinducedoral mucositis", INTERNET, 24 September 2013 (2013-09-24), Retrieved from the Internet: URL:http://www.omicsgroup.com/conferences/ cancer-science-therapy-2013/
- MENOTTI C: "Mucosamin spray", INTERNET, 4 November 2010 (2010-11-04), pages 1-4, Retrieved from the Internet: URL:http://www.solartium-dietetics.com/sol artium/sites/default/files/u9/mucosamin.pd f & Mariantonietta Soloperto: "Prevenzione e gestione di mucosite, dolore ed emesi", 10 anni di Radioterapia a Taranto; Taranto; 15 dicembre 2012 10 anni di Radioterapia a Taranto; Taranto; 15 dicembre 2012, 15 December 2012 (2012-12-15), pages 1-29, Retrieved from the Internet: URL:http://www.congressiairo.it/2012/taran to10anni/SOLOPERTO.pdf [retrieved on 2014-05-12]
- L Kaynar, Cetin A, Hacioglu Sk, Eser B, Koçyigit I, Canöz Ö, Tasdemir A, Karadag C, Kurnaz F, Saraymen R, Silici S: "EFFICACY OF ROYAL JELLY ON METHOTREXATE-INDUCED SYSTEMIC OXIDATIVE STRESS AND DAMAGE TO SMALL INTESTINE IN RATS", African Journal of Traditional, Complementary and Alternative Medicines, vol. 9, no. 3, 2012, pages 412-417,
- Khanal B; Baliga M; Uppal N: "Effect of topical honey on limitation of radiation-induced oral mucositis: an intervention study", INTERNATIONAL JOURNAL OF ORAL AND MAXILLOFACIAL SURGERY,, vol. 39, no. 12, December 2010 (2010-12), pages 1181-1185,
- U M Rashad, S M Al-Gezawy, E El-Gezawy, A N Azzaz: "Honey as topical prophylaxis against radiochemotherapy-induced mucositis in head and neck cancer", The Journal of Laryngology & Otology, vol. 123, no. 2, February 2009 (2009-02), pages 223-228,
- Motallebnejad M, Akram S, Moghadamnia A, Moulana Z, Omidi S.: "The effect of topical application of pure honey on radiation-induced mucositis: a randomized clinical trial", J Contemp Dent Pract., vol. 9, no. 3, 1 March 2008 (2008-03-01), pages 40-47,
- Biswal BM, Zakaria A, Ahmad NM.: "Topical application of honey in the management of radiation mucositis: A preliminary study", Support Care Cancer, vol. 11, no. 4, April 2003 (2003-04), pages 242-248,
- HERMOSIN et al.: "Free amino acid composition and botanical origin of honey", Food Chemistry, vol. 83, 2003, pages 263-268,
- PICCIRILLO et al.: "Glutamine-enriched parenteral nutrition after autologous peripheral blood stem cell transplantation: effects on immune reconstitution and mucositis", Haematologica, vol. 88, 2003, pages 192-200,
- ANDERSON et al.: "Effect of low-dose oral glutamine on painful stomatitis during bone marrow transplantation", Bone Marrow Transplantation, vol. 22, no. 4, 4 August 1998 (1998-08-04) , pages 339-344,
- FAVIA et al.: "Accelerated wound healing of oral soft tissues and angiogenic effect induced by a pool of aminoacids combined to sodium hyaluronate (AMINOGAM (R))", JOURNAL OF BIOLOGICAL REGULATORS AND HOMEOSTATIC AGENTS, vol. 22, no. 2, 2008, pages 109-116,
- Decision Opposition Division dated 10.11.2017 (12703533.5/2811845)
- D'HONDT et al.: "Oral mucositis induced by anticancer treatments: physiopathology and treatments", Therapeutics and Clinical Risk Management,, vol. 2, no. 2, 2006, pages 159-168,
- Declaration from Prof. N. Cirillo, 18 June 2014.

## Description

The present invention relates to a composition comprising glycine, proline, a natural or synthetic film-forming polymer, lysine and leucine, for use in the prevention of oral mucositis induced by radiation or chemotherapy, wherein the composition is to be administered orally.

### Technical background

Oral mucositis (OM) is one of the most frequent and potentially serious side effects of non-surgical antitumoral treatment (1,2). Clinically, it is characterised by erythema, formation of ulcers with or without pseudo-membranes, bleeding, formation of exudates, and/or infections located at the upper level (3), and can evolve into a debilitating form which adversely affects the patient's quality of life. The damage to the mucous membranes can extend from the oral cavity to the pharynx; in this invention, therefore, the term "oral mucositis" includes mucositis of the pharynx.

The patient's perception of the symptoms associated with OM range from a sensation of pain in the mouth to impairment of various oral functions (4). OM can be associated with pain symptoms such as dysgeusia, dysphagia, and difficulty in speaking, chewing or swallowing, with consequent impairment of the ability to eat, weight loss, and the need for feeding through a nasogastric tube.

All this not only has an adverse impact on the patient's quality of life, but can also lead to suspension of the treatment or a reduction in the dose of antitumoral medicament, with a consequent decline in the patient's life expectancy (5). In financial terms, the frequent hospital admissions of patients suffering from OM, and their home care by personnel skilled in the administration of drugs or parenteral feeding, involve a considerable burden on the national health service (6,7).

Despite the severity and frequency of cases of OM and many studies conducted on the subject, there is still no universally accepted standard strategy for the prevention and treatment of this potentially serious complication. Although some trials have demonstrated that the onset of OM can be prevented or reduced, they were conducted on an insufficient number of participants, and do not meet adequate standards which allow the drafting of clinical practice guidelines (8-11).

At present, the treatment of OM and the pain caused by it comprise basic topical anaesthetics, systemic analgesics, anti-inflammatory, mucosa coating agents, mouthwashes, oral hygiene in general, and antimicrobials (5,12-15). A number of other agents have also been tested, such as growth factors and cytokines, biological compounds that protect the mucosa, cryotherapy, and terapy with low-level laser (13,16). Hyaluronic acid (HA) plays an important role in tissue healing, by means of various mechanisms (17-19); clinical trials have also confirmed that it improves the healing process of ulcers of the lower limbs (20) and the nasal mucosa after surgery (21) and reduces acute epithelitis in patients who undergo radiotherapy to treat head, neck, breast or pelvic cancer (22).

The research therefore focuses on new treatment and prevention strategies for oral mucositis and the corresponding formulations.

It was recently demonstrated that a gel based on 0.2% HA for topical use not only relieves the pain/burning sensation associated with recurrent aphthous stomatitis and atrophic/erosive oral lichen planus, but also contributes to the clinical resolution of areas presenting ulcers, erosions or atrophy (23, 24).

International patent application WO2007/048522 discloses wound-healing pharmaceutical compositions comprising a combination of glycine, lysine, leucine and proline and sodium hyaluronate, which is particularly effective in facilitating the cell renewal process that forms the basis of rapid wound-healing, promoting connective tissue reconstruction and consequent regeneration of the epithelial cells.

The same combination has also proved able to treat oral and vaginal or rectal mucositis (WO2011/064297).

LIONEL D'HONDT ET AL: "Oral mucositis induced by anticancer treatments: physiopathology and treatments." THERAPEUTICS AND CLINICAL RISK MANAGEMENT JUN 2006, discloses many candidates for prevention of radiotherapy-induced mucositis.

However, the problem of preventing mucositis remains unsolved. An agent able to prevent the onset of mucositis when administered before chemotherapy or radiotherapy treatment would represent significant medical progress, improving the quality of life of cancer patients.

### Description of the invention

It has now been found that compositions comprising glycine, proline, a natural or synthetic film-forming polymer, lysine and leucine, are effective in the prevention/prophylaxis of oral mucositis induced by radiation or chemotherapy, when administered before the start of the chemotherapy or radiotherapy treatment.

The compositions according to the present invention have proved able to prevent the onset of mucositis when administered at least 24 hours and up to 15 days before the start of pharmacological or therapeutic treatment liable to induce inflammation of the mucous membranes.

The present invention therefore relates to a composition comprising:
a) glycine,
b) proline,
c) a natural or synthetic film-forming polymer,
d) lysine, and
e) leucine,
for use in the prevention of oral mucositis induced by radiation or chemotherapy, wherein the composition is to be administered orally.

According to the invention, the natural or synthetic film-forming polymer is selected from hyaluronic acid or a salt thereof and polyvinylpyrrolidone.

According to a preferred aspect, the natural or synthetic film-forming polymer is hyaluronic acid or a salt thereof.

According to the invention, the amino acids are present in the L form.

According to a preferred aspect, the compositions according to the invention will contain the various active ingredients in the following composition ranges by weight:
a) 0.5 to 20% glycine,
b) 0.2 to 15% proline,
c) 0.5 to 5% hyaluronic acid or a salt thereof,
d) 0.05 to 10% lysine, and
e) 0.05% to 3% leucine.

The compositions according to the invention can be formulated suitably for oral topical administration in the form of a spray, aerosol, mouthwash or gel, and will be prepared according to conventional methods well known in pharmaceutical technology, such as those described in Remington's Pharmaceutical Handbook, Mack Publishing Co., N.Y., USA, using excipients, solubilisers, emollients, stabilisers, emulsifiers, pH regulators and preservatives acceptable for their final use.

Pharmacological experiments have demonstrated the ability of the compositions according to the invention to prevent the oxidative action of ROS on fibroblast aging, which leads to a reduction in the metabolic activity of the keratinocytes.

Its efficacy has also been confirmed by clinical trials conducted on a statistically significant sample of patients suffering from tumours of various origins who were due to undergo chemotherapy (doxorubicin, cisplatin) and/or radiotherapy cycles. Some of the patients recruited to the trial had a prior history of mucositis of varying degrees following chemotherapy treatment.

The patients were treated three times a day for four consecutive days, starting four days before the chemotherapy treatment, with a spray containing hyaluronic acid, glycine, proline, lysine and leucine (Mucosamin®). The treatment was continued throughout the treatment cycle. The composition was delivered in quantities amounting to three puffs, and was maintained *in situ* for at least 2 minutes. Patients were told not to eat, drink or rinse their mouths for at least one hour thereafter.

The patients were evaluated with a physical examination at T#0 (first examination and start of prophylaxis), T#1 (start of antitumoral treatment), T#2 (check-up after 24 hours), T#3 (check-up after 72 hours), T#4 (check-up after 7 days), T#5 (check-up after 14 days) and end of treatment. In 80% of cases there was no onset of mucositis, and in the remaining 20% only mild mucositis (grade 1) was observed.

### REFERENCES

1. Scully C et al., Oral Dis 2006; 12:229-241.
2. Scully C, Epstein J, Sonis S. Oral mucositis: a challenging complication of radiotherapy, chemotherapy, and radiochemotherapy: part 1, pathogenesis and prophylaxis of mucositis. Head Neck 2003; 25:1057-1070.
3. Compilato D et al., J Oral Pathol Med 2009; 38:241-253.
4. Scully C et al., Oral mucositis: a challenging complication of radiotherapy, chemotherapy, and radiochemotherapy. Part 2: diagnosis and management of mucositis. Head Neck 2004; 26:77-84.
5. Saadeh CE. Pharmacotherapy 2005; 25:540-554.
6. Peterman A et al., J Natl Cancer Inst Monogr 2001:45-51.
7. Elting LS, et al., Cancer 2003; 98:1531-1539.
8. Rubenstein EB et al., Cancer 2004; 100:2026-2046.
9. Worthington HV et al., Cochrane Database Syst Rev 2007:CD000978.
10. Clarkson JE et al., Cochrane Database Syst Rev 2007:CD001973.
11. Potting C et al., J Clin Nurs 2009; 18:3-12.
12. Epstein JB et al., Oncology (Williston Park) 2003; 17:1767-1779; discussion 1779-1782, 1791-1762.
13. Bensadoun RJ et al., Bull Cancer 2006; 93:201-211.
14. Bensinger W et al., J Natl Compr Canc Netw 2008; 6 Suppl 1 :S1-21; quiz S22-24.
15. Lalla RV et al., Dent Clin North Am 2008; 52:61-77.
16. Arora H, et al., Oral Surg Oral Med Oral Pathol Oral Radiol Endod 2008; 105:180-186.
17. Chen WY, et al., Wound Repair Regen 1999; 7:79-89.
18. Price RD, et al., J Plast Reconstr Aesthet Surg 2007; 60:1110-1119.
19. David-Raoudi et al., Wound Repair Regen 2008; 16:274-287.
20. Ortonne JP. Ann Dermatol Venereol 2001; Suppl: 13-16.
21. Soldati D, et al., Drugs Exp Clin Res 1999; 25:253-261.
22. Liguori V et al., Radiother Oncol 1997; 42:155-161.
23. Nolan A et al., J Oral Pathol Med 2009; 38:299-303.
24. Nolan A, et al., J Oral Pathol Med 2006; 35:461-465.

## Claims

1. A composition comprising:
a) glycine,
b) proline,
c) a natural or synthetic film-forming polymer,
d) lysine, and
e) leucine,
for use in the prevention of oral mucositis induced by radiations or chemotherapeutics, wherein the composition is to be administered orally.

2. A composition for use according to claim 1, wherein the natural or synthetic film-forming polymer is selected from hyaluronic acid or a salt thereof and polyvinylpyrrolidone.

3. A composition for use according to claim 2, wherein the natural or synthetic film-forming polymer is hyaluronic acid or a salt thereof.

4. A composition for use according to claim 1, wherein the amino acids are in the L form.

5. A composition for use according to claim 1 and 2, wherein the various components of the composition are present in the following ranges by weight:
a) 0.5 to 20% of glycine,
b) 0.2 to 15% of proline,
c) 0.5 to 5% of hyaluronic acid or a salt thereof,
d) 0.05 to 10% of lysine, and
e) 0.05% to 3% of leucine.

6. A composition for use according to claim 1 and 2, wherein the composition is in the form of a spray, aerosol, mouthwash or gel.

## Patentansprüche

1. Zusammensetzung, umfassend:
a) Glycin;
b) Prolin;
c) ein natürliches oder synthetisches filmbildendes Polymer;
d) Lysin; und
e) Leucin,
zur Verwendung bei der Prävention einer oralen Mucositis, die durch Strahlungen oder Chemotherapeutika induziert ist, wobei die Zusammensetzung oral zu verabreichen ist.

2. Zusammensetzung zur Verwendung gemäß Anspruch 1, wobei das natürliche oder synthetische filmbildende Polymer aus Hyaluronsäure oder einem Salz davon und Polyvinylpyrrolidon ausgewählt ist.

3. Zusammensetzung zur Verwendung gemäß Anspruch 2, wobei es sich bei dem natürlichen oder synthetischen filmbildenden Polymer um Hyaluronsäure oder ein Salz davon handelt.

4. Zusammensetzung zur Verwendung gemäß Anspruch 1, wobei die Aminosäuren in der L-Form vorliegen.

5. Zusammensetzung zur Verwendung gemäß Anspruch 1 und 2, wobei die verschiedenen Komponenten der Zusammensetzung in den folgenden Gewichtsbereichen vorhanden sind:
a) 0,5 bis 20% Glycin,
b) 0,2 bis 15% Prolin,
c) 0,5 bis 5% Hyaluronsäure oder ein Salz davon;
d) 0,05 bis 10% Lysin; und
e) 0,05 bis 3% Leucin.

6. Zusammensetzung zur Verwendung gemäß Anspruch 1 und 2, wobei die Zusammensetzung in der Form eines Sprays, Aerosols, einer Mundspülung oder eines Gels vorliegt.

## Revendications

1. Composition comprenant :
a) de la glycine,
b) de la proline,
c) un polymère filmogène naturel ou synthétique,
d) de la lysine, et
e) de la leucine,
pour l'utilisation dans la prévention de l'inflammation d'une muqueuse buccale induite par les rayonnements ou les chimiothérapeutiques, dans laquelle la composition doit être administrée par voie orale.

2. Composition pour l'utilisation selon la revendication 1, dans laquelle le polymère filmogène naturel ou synthétique est choisi parmi l'acide hyaluronique ou un sel de celui-ci et la polyvinylpyrrolidone.

3. Composition pour l'utilisation selon la revendication 2, dans laquelle le polymère filmogène naturel ou synthétique est l'acide hyaluronique ou un sel de celui-ci.

4. Composition pour l'utilisation selon la revendication 1, dans laquelle les acides aminés sont sous la forme L.

5. Composition pour l'utilisation selon la revendication 1 et la revendication 2, dans laquelle les divers composants de la composition sont présents en les intervalles en poids suivants :
a) 0,5 à 20 % de glycine,
b) 0,2 à 15 % de proline,
c) 0,5 à 5 % d'acide hyaluronique ou d'un de ses sels,
d) 0,05 à 10 % de lysine, et
e) 0,05 % à 3 % de leucine.

6. Composition pour l'utilisation selon la revendication 1 et la revendication 2, la composition étant sous la forme d'un spray, d'un aérosol, d'un bain de bouche ou d'un gel.
